# EUROPEAN PATENT APPLICATION

(11) **EP 4 825 436 A1**
(43) Date of publication of application: **21.10.2026**
(21) Application number: 24903742.5
(22) Date of filing: 12.12.2024
(51) Int. Cl.: A61N 5/067, A61B 18/20

(54) **LIGHT IRRADIATION DEVICE AND LIGHT IRRADIATION SYSTEM**

(30) Priority: 15.12.2023 JP 2023211864; 15.12.2023 JP 2023211865
(71) Applicant: NICHIA CORPORATION, Anan-shi, Tokushima 774-8601 (JP)
(72) Inventor: TSUKAMOTO, Toshihiko, Owariasahi-shi Aichi 488-0061 (JP); MIYATA, Tadaaki, Anan-shi, Tokushima 774-8601 (JP)
(74) Representative: Betten & Resch
(86) International application number: PCT/JP2024/043931
(87) International publication number: WO 2025/127089

(57) **Abstract**

The light irradiation device is a light irradiation device for medical use having an elongated shape, and includes a device body, a laser light source, and a cooling liquid passage. The device body has an elongated shape. The laser light source is provided at a distal end portion of the elongated device body and emits laser light. The cooling liquid passage extends to the laser light source in the device body, and supplies a cooling liquid for cooling the laser light source to the laser light source.

## Description

### [Technical Field]

The present disclosure relates to a light irradiation device and a light irradiation system that are inserted into a biological lumen and irradiate light.

### [Background Art]

As one of the techniques for treating diseases, PDT (Photodynamic Therapy) has been known. In PDT, after a photosensitizing substance is administered to a living body, light is irradiated onto the living body. As a result, cancer cells may be destroyed by reactive oxygen species generated in the cancer cells. However, in PDT, it is difficult to selectively accumulate the photosensitizing substance in cancer cells. The occurrence of side effects resulting from uptake of the photosensitizing substance by normal cells can be a problem in PDT.

In contrast, in recent years, NIR-PIT (Near-infrared photoimmunotherapy) has been proposed. In NIR-PIT, a complex formed by binding two compounds, namely an antibody against an antigen specific to cancer cells and a photosensitizing substance, is used. When administered to a living body, the complex tends to selectively accumulate in cancer cells in the body. Thereafter, the complex is activated by irradiation with light having the excitation wavelength of the photosensitizing substance in the complex (for example, a wavelength including 690 nm) (see, for example, Patent Literature 1). In NIR-PIT, when the complex is selectively accumulated in cancer cells by the antibody and light is locally irradiated onto the cancer cells, side effects are less likely to occur than in PDT.

Light in a wavelength range including 690 nm hardly penetrates into the body even when irradiated from the body surface, and therefore it is difficult to treat cancer located deep within the body by irradiation from the body surface. Accordingly, a technique has been proposed for irradiating light not from the body surface but from a position closer to the cancer cells. For example, the device described in Patent Literature 2 irradiates light from deep within the body by being inserted into a blood vessel.

### [Prior Art Documents]

### [Patent Literatures]

[Patent Literature 1] JP 2014-523907 A
[Patent Literature 2] JP 2018-000867 A

### [Summary of the Invention]

In conventional devices, it is necessary to transmit light emitted from a light source provided externally to a distal end of the device by a light transmission member (for example, an optical fiber or the like). When the light transmission member is used, bending or the like may occur in the light transmission member within a body lumen, and the light may leak or attenuate before being transmitted to the distal end of the device. If the light leaks or attenuates, problems such as a decrease in the transmission efficiency of the light or a decrease in safety may occur. Further, in the process in which the light is transmitted by the light transmission member, characteristics of the light (for example, wavelength or the like) may change, and the intended therapeutic effect may become difficult to obtain. Therefore, it is desirable to use a laser light source that can be inserted into the body without using a light transmission member. However, when the laser light source is used by being inserted into the body, unless the heat dissipation of the laser light source is improved while miniaturizing the laser light source, problems caused by a temperature rise (for example, failure of the laser light source or the like) may occur.

A typical objective of the present disclosure is to provide a light irradiation device and a light irradiation system capable of irradiating light onto a specific position within a body lumen more efficiently and appropriately.

A light irradiation device provided by a typical embodiment of the present disclosure is a light irradiation device for medical use having an elongated shape, including: a laser light source disposed at a distal end portion of an elongated device body and configured to emit laser light; and a cooling liquid passage extending to the laser light source in the device body and supplying a cooling liquid for cooling the laser light source to the laser light source.

A light irradiation system provided by a typical embodiment of the present disclosure is a light irradiation system for medical use including: a catheter formed in an elongated tubular shape; and an elongated light irradiation device inserted into a lumen of the catheter, wherein the light irradiation device includes: a laser light source disposed at a distal end portion of an elongated device body and configured to emit laser light; and a cooling liquid passage extending to the laser light source in the device body and supplying a cooling liquid for cooling the laser light source to the laser light source, and a light-transmitting portion configured to transmit, to an outside of the catheter, laser light emitted by the laser light source in the light irradiation device is formed in at least a part of a distal end portion of the catheter.

According to the light irradiation device and the light irradiation system according to the present disclosure, light is irradiated more efficiently and appropriately to a specific position within a lumen of a living body.

### (First Aspect)

The light irradiation device of the present disclosure is a medical light irradiation device having an elongated shape, and includes a device body, a laser light source, and a cooling liquid passage. The device body has an elongated shape. The laser light source is provided at a distal end portion of the elongated device body, and emits laser light. The cooling liquid passage extends to the laser light source in the device body, and supplies a cooling liquid for cooling the laser light source to the laser light source.

According to the light irradiation device of the present disclosure, a temperature rise of the laser light source and the vicinity thereof is appropriately suppressed by the cooling liquid supplied to the laser light source through the cooling liquid passage. Accordingly, problems caused by a temperature rise of the distal end portion due to the laser light source (for example, at least one of failure of the laser light source and coagulation of blood) are appropriately suppressed. The cooling liquid passage only needs to extend to the laser light source to such an extent that the cooling liquid can be supplied to the laser light source. Accordingly, the distal end portion of the cooling liquid passage does not necessarily have to reach the position of the laser light source or a position further toward the distal end than the laser light source.

The laser light source may emit the laser light in a direction intersecting the longitudinal axis direction of the light irradiation device. In this case, the light irradiation device can selectively irradiate a specific position in a living body with the laser light emitted from the laser light source.

The device body may be formed in an elongated tubular shape, so that a cooling liquid passage may be defined in a lumen of the device body. When an area of the cooling liquid passage in a cross-section taken along a direction orthogonal to the extending direction of the device body is defined as a passage area, the passage area at a portion of the cooling liquid passage on the proximal side of the laser light source may be larger than the passage area at a portion of the cooling liquid passage where the laser light source is disposed.

In this case, pressure loss of the cooling liquid is less likely to occur in a path to the vicinity of the laser light source in the device, and the cooling liquid is more likely to appropriately flow through the vicinity of the laser light source. Accordingly, the laser light source can be cooled more easily and efficiently. The range in which the passage area is made larger than the passage area at the portion where the laser light source is disposed (that is, the range of the "portion on the proximal side of the laser light source") can be appropriately set. For example, the passage area throughout the entire range extending from the proximal end of the laser light source itself toward the proximal side may be larger than the passage area at the portion where the laser light source is disposed. Further, a certain distance (for example, a distance equal to or less than a length L when a length of the laser light source in an axial direction of the device body is defined as L) may be provided between the range in which the passage area is made larger than the passage area at the portion where the laser light source is disposed and the proximal end of the laser light source itself.

A specific method for making the passage area at the portion where the laser light source is disposed smaller than the passage area at an adjacent portion on the proximal side of the laser light source can be appropriately selected. For example, while keeping the inner diameter of the cooling liquid passage constant, the cross-sectional area of a member adjacent on the proximal side of the laser light source may be made smaller than the cross-sectional area of a member where the laser light source is disposed. In this case, the passage area at the portion where the laser light source is disposed can be made smaller than the passage area at an adjacent portion on the proximal side of the laser light source while the shape of the cooling liquid passage is simplified. The inner diameter of the cooling liquid passage at the portion where the laser light source is disposed may be smaller than the inner diameter of the cooling liquid passage at an adjacent portion on the proximal side of the laser light source. In this case, the passage area at the portion where the laser light source is disposed can be made smaller than the passage area at an adjacent portion on the proximal side of the laser light source while reducing the diameter of the light irradiation device in the vicinity of the laser light source.

The light irradiation device may further include a power supply line. The power supply line extends from the proximal end side to the distal end side of the device body and is connected to the laser light source so as to supply electric power to the laser light source. The power supply line may be exposed in the cooling liquid passage. In this case, heat generated from the laser light source is more likely to be released to the cooling liquid through the power supply line. Accordingly, the laser light source can be cooled more easily and efficiently.

In the power supply line, the cross-sectional area of a connecting portion of the power supply line where the power supply line is connected to the laser light source may be larger than the cross-sectional area of a portion of the power supply line on the proximal side of the connecting portion. In this case, heat generated from the laser light source is more likely to propagate to the power supply line as compared with a case where the cross-sectional area of the power supply line is constant or the like. Accordingly, the laser light source can be cooled more easily and efficiently.

At least a part of the surface of the power supply line may be covered with an insulating material. The thickness of the insulating material covering the power supply line may be smaller than the thickness of the power supply line without being covered with the insulating material. In this case, as compared with a case where the thickness of the insulating material is equal to or greater than the thickness of the power supply line, heat propagated from the laser light source to the power supply line is more likely to be further released to the outside through the insulating material. Accordingly, the laser light source can be cooled more easily and efficiently.

The thickness of the insulating material covering the power supply line may more preferably be 25% or less of the thickness of the power supply line, and still more preferably 10% or less. In this case, heat propagated from the laser light source to the power supply line is more likely to be further released to the outside.

The light irradiation device may further include a supply detection unit configured to detect whether cooling liquid is being supplied into the cooling liquid passage, and a supply notification unit configured to provide notification of the detection result by the supply detection unit. In this case, the user can appropriately ascertain whether or not cooling liquid is being supplied into the cooling liquid passage. Therefore, various malfunctions caused by emission of laser light while cooling liquid is not supplied are less likely to occur.

Note that the specific configuration of the supply detection unit can be selected as appropriate. For example, the light irradiation device may include a passage valve that prevents backflow of cooling liquid toward the proximal side of the cooling liquid passage (details will be described later). The supply detection unit may detect whether or not cooling liquid is being supplied into the cooling liquid passage by detecting whether or not the passage valve is open. In this case, the presence or absence of cooling liquid supply is appropriately detected according to whether or not the passage valve is open. The supply detection unit may also be a flow rate sensor or the like that detects the flow of cooling liquid by being provided in at least a part of the passage of the cooling liquid. It is also possible to use a temperature sensor as the supply detection unit. In this case, the temperature sensor may also be used both for detection of the ambient temperature and for detection of the presence or absence of cooling liquid supply.

The light irradiation device may further include a temperature detection unit configured to detect the temperature of the laser light source (or the vicinity of the laser light source), and a temperature notification unit configured to provide notification of the detection result by the temperature detection unit. In this case, the user can easily ascertain whether or not the temperature of the laser light source is being appropriately maintained.

Note that the specific configuration of the temperature detection unit can be selected as appropriate. For example, the temperature detection unit may be disposed in the laser light source. The temperature detection unit may also detect the temperature in the vicinity of the laser light source (for example, cooling liquid or the like in the vicinity of the laser light source). The temperature of the laser light source may also be detected to be at or below a threshold by monitoring the state of the laser light source.

The light irradiation device may further include a passage valve. The passage valve is provided on a proximal end side of the laser light source in the cooling liquid passage, and prevents backflow of the cooling liquid toward the proximal side of the cooling liquid passage. By providing the passage valve, backflow of the cooling liquid in the cooling liquid passage is prevented. As a result, it becomes easier to more appropriately suppress a temperature rise of the laser light source and the vicinity thereof.

The light irradiation device may further include a Peltier element provided at a distal end portion of the device body. The Peltier element may be arranged in contact with or close to the laser light source and have a cooling surface facing the laser light source. By providing the Peltier element, problems caused by a temperature rise of the distal end portion due to the laser light source are appropriately suppressed by the cooling effect of the element.

Note that, when the Peltier element is disposed close to the laser light source, the cooling surface of the Peltier element and the laser light source may be separated from each other to such an extent that the cooling effect of the Peltier element on the laser light source can be appropriately obtained. Further, the cooling surface of the Peltier element and the laser light source may be close to each other in a state where at least one of an adhesive and a member having a high thermal conductivity or the like is disposed between the cooling surface of the Peltier element and the laser light source. Even in this case, if the cooling surface of the Peltier element and the laser light source are thermally connected, the cooling effect of the Peltier element on the laser light source can be appropriately obtained.

An outer periphery of the Peltier element may be covered with a covering member (for example, resin or the like) that prevents liquid from entering an interior thereof. In this case, problems such as failure of the Peltier element due to ingress of liquid are appropriately suppressed.

Of the Peltier element, at least one of a heating surface located on a side opposite to the cooling surface and an indirect heat-dissipating member having a thermal conductivity equal to or greater than that of the heating surface and being in contact with the heating surface may be externally exposed to a liquid (for example, a cooling liquid or the like). In this case, compared with a case where the heating surface is not directly or indirectly exposed, heat generated from the heating surface of the Peltier element is more likely to be appropriately dissipated.

Note that a member having a high thermal conductivity (for example, at least one of a heat pipe, carbon nanotubes, ceramics (AIN, SiC, etc.), a metal plate (platinum, titanium, copper), and a torque wire or the like) may be disposed on the heating surface of the Peltier element. In this case, heat generated from the heating surface of the Peltier element is likely to be dissipated even more efficiently. Note that, when a metal plate is used, the metal plate may be an alloy, or the surface of the metal plate may be plated.

Instead of the Peltier element, a member having a high thermal conductivity (for example, at least one of a heat pipe, carbon nanotubes, ceramics (AIN, SiC, etc.), a metal plate (platinum, titanium, copper), and a torque wire or the like) may be arranged in contact with or in proximity to the laser light source. Also in this case, the heat emitted from the laser light source is likely to be appropriately dissipated. As described above, when a metal plate is used, the metal plate may be an alloy, or the surface of the metal plate may be plated.

The light irradiation system of the present disclosure is a light irradiation system for medical use, and includes a catheter formed in an elongated tubular shape, and an elongated light irradiation device inserted into a lumen of the catheter. The light irradiation device includes a laser light source and a cooling liquid passage. The laser light source is disposed at a distal end portion of an elongated device body and emits laser light. The cooling liquid passage extends to the laser light source in the device body and supplies a cooling liquid for cooling the laser light source to the laser light source. At least a part of the distal end portion of the catheter is formed with a light-transmitting portion that transmits, to the outside of the catheter, laser light emitted by the laser light source provided in the light irradiation device.

According to the light irradiation system of the present disclosure, a rise in temperature of the laser light source and the vicinity thereof is appropriately suppressed by the cooling liquid supplied to the laser light source through the cooling liquid passage. Therefore, problems caused by a rise in temperature of the distal end portion due to the laser light source (for example, at least one of failure of the laser light source and coagulation of blood) are appropriately suppressed. Furthermore, the light emitted from the laser light source passes through the light-transmitting portion of the catheter and is irradiated onto biological tissue. Thus, light is irradiated more efficiently and appropriately to a specific position within a body lumen.

At least a part of the distal end portion of the catheter may be formed with a discharge port for discharging, to the outside of the catheter, the cooling liquid discharged from the cooling liquid passage of the light irradiation device. In this case, the cooling liquid supplied through the cooling liquid passage of the light irradiation device passes through the vicinity of the laser light source and is discharged to the outside from the discharge port of the catheter. As a result, since the cooling liquid continues to be supplied to the vicinity of the laser light source, a rise in temperature of the laser light source and the vicinity thereof can be further appropriately suppressed. Further, the possibility that blood outside the catheter comes into contact with the laser light source is also appropriately reduced.

A specific configuration of the discharge port may be selected as appropriate. For example, the distal end portion of the catheter may be provided with a passage hole through which a guide wire is inserted. The passage hole for the guide wire may also serve as the discharge port for the cooling liquid. Further, separately from the passage hole for the guide wire, a discharge port for the cooling liquid may be formed in the distal end portion of the catheter.

A discharge valve may be provided at the discharge port of the catheter, the discharge valve allowing discharge of liquid to the outside of the catheter through the discharge port while preventing inflow of liquid into the catheter from the outside. In this case, the discharge valve suppresses blood or the like outside the catheter from flowing into the catheter through the discharge port. As a result, the possibility that blood comes into contact with the laser light source is further reduced.

### (Second Aspect)

The light irradiation device of the present disclosure is a light irradiation device for medical use having an elongated shape, and includes a device body, a laser light source, and a Peltier element. The device body has an elongated shape. The laser light source is disposed at a distal end portion of the elongated device body and emits laser light. The Peltier element is provided at the distal end portion of the device body. The Peltier element is arranged in contact with or in proximity to the laser light source and has a cooling surface facing the laser light source.

According to the light irradiation device of the present disclosure, problems caused by a temperature rise in the distal end portion due to the laser light source are appropriately suppressed by the cooling effect of the Peltier element.

In a case where the Peltier element is arranged in proximity to the laser light source, the cooling surface of the Peltier element and the laser light source may be spaced apart from each other to such an extent that the cooling effect of the Peltier element on the laser light source can be appropriately obtained. Further, the cooling surface of the Peltier element and the laser light source may be in proximity to each other in a state in which at least one of an adhesive and a member having a high thermal conductivity or the like is disposed between the cooling surface of the Peltier element and the laser light source. Even in this case, if the cooling surface of the Peltier element and the laser light source are thermally connected, the cooling effect of the Peltier element on the laser light source can be appropriately obtained.

An outer periphery of the Peltier element may be covered with a covering member (for example, resin or the like) that prevents liquid from entering the interior. In this case, problems such as failure of the Peltier element due to ingress of liquid are appropriately suppressed.

The laser light source may emit laser light in a direction intersecting the longitudinal direction of the light irradiation device. In this case, the light irradiation device can selectively irradiate a specific position in a living body with the laser light emitted from the laser light source.

In the Peltier element, at least one of a heating surface located on the side opposite to the cooling surface and an indirect heat-dissipating member that is in contact with the heating surface and has a thermal conductivity equal to or higher than that of the heating surface may be exposed externally to a liquid (for example, a cooling liquid or the like). In this case, as compared with a case where the heating surface is not directly or indirectly exposed, heat emitted from the heating surface of the Peltier element is more readily and appropriately dissipated.

A member having a high thermal conductivity (for example, at least one of a heat pipe, a carbon nanotube, ceramics (AIN, SiC, etc.), a metal plate (platinum, titanium, copper), and a torque wire) may be provided on the heating surface of the Peltier element. In this case, heat emitted from the heating surface of the Peltier element can be dissipated more readily and efficiently. When a metal plate is used, the metal plate may be an alloy, and the surface of the metal plate may be plated.

Instead of the Peltier element, a member having a high thermal conductivity (for example, at least one of a heat pipe, a carbon nanotube, ceramics (AIN, SiC, etc.), a metal plate (platinum, titanium, copper), and a torque wire) may be disposed in contact with or in proximity to the laser light source. Also in this case, heat emitted from the laser light source is more readily and appropriately dissipated. As described above, when a metal plate is used, the metal plate may be an alloy, and the surface of the metal plate may be plated.

The light irradiation device may further include a cooling liquid passage. The cooling liquid passage extends to the laser light source in the device body, and supplies a cooling liquid for cooling at least one of the laser light source and the Peltier element to the laser light source. In this case, a temperature rise of the laser light source and the vicinity thereof is appropriately suppressed by the cooling liquid that passes through the cooling liquid passage and is supplied to the laser light source side. Accordingly, problems caused by a temperature rise in the distal end portion due to the laser light source (for example, at least one of failure of the laser light source and coagulation of blood) are appropriately suppressed. The cooling liquid passage need only extend to the laser light source to such an extent that the cooling liquid can be supplied to the laser light source. Accordingly, the distal end portion of the cooling liquid passage does not necessarily have to reach the laser light source.

When the device body is formed in a long tubular shape, a cooling liquid passage may be defined in the lumen of the device body. In a cross section taken along a direction intersecting the extending direction of the device body perpendicularly, when an area of the cooling liquid passage is defined as a passage area, the passage area of a portion of the cooling liquid passage adjacent to the laser light source on the proximal side may be larger than the passage area at a portion of the cooling liquid passage where the laser light source is disposed.

In this case, pressure loss of the cooling liquid in a path to the vicinity of the laser light source inside the device is less likely to occur, and the cooling liquid is more likely to appropriately flow through the vicinity of the laser light source. Accordingly, the laser light source can be cooled more efficiently. The range in which the passage area is made larger than the passage area at the portion where the laser light source is disposed (that is, the range of the "portion on the proximal side of the laser light source") can be set as appropriate. For example, the passage area of the entire range extending from the proximal end of the laser light source itself toward the proximal end side may be made larger than the passage area at the portion where the laser light source is disposed. Further, a certain distance (for example, when a length of the laser light source in an axial direction of the device body is L, a distance equal to or less than the length L) may be provided between the range in which the passage area is made larger than the passage area at the portion where the laser light source is disposed and the proximal end of the laser light source itself.

The specific method for making the passage area at the portion where the laser light source is disposed smaller than the passage area at an adjacent portion on the proximal side of the laser light source can be selected as appropriate. For example, while keeping the inner diameter of the cooling liquid passage constant, the cross-sectional area of a portion adjacent to the laser light source on the proximal side may be made smaller than the cross-sectional area of the portion where the laser light source is disposed. In this case, the passage area at the portion where the laser light source is disposed can be made smaller than the passage area at an adjacent portion on the proximal side of the laser light source, with the shape of the cooling liquid passage simplified. Further, the inner diameter of the cooling liquid passage at the portion where the laser light source is disposed may be made smaller than the inner diameter of the cooling liquid passage at an adjacent portion on the proximal side of the laser light source. In this case, the passage area at the portion where the laser light source is disposed can be made smaller than the passage area at an adjacent portion on the proximal side of the laser light source while reducing the diameter of the light irradiation device in the vicinity of the laser light source.

The light irradiation device may further include a power supply line. The power supply line extends from the proximal end side to the distal end side of the device body and supplies electric power to the laser light source by being connected to the laser light source. The power supply line may be exposed in the cooling liquid passage. In this case, heat generated from the laser light source is more likely to be released to the cooling liquid through the power supply line. Accordingly, the laser light source can be cooled more efficiently.

Of the power supply line, the cross-sectional area of a connecting portion where the power supply line is connected to the laser light source may be larger than the cross-sectional area of a portion of the power supply line on the proximal side of the connecting portion. In this case, as compared with, for example, a case where the cross-sectional area of the power supply line is constant, heat generated from the laser light source is more likely to be transmitted to the power supply line. Therefore, the laser light source can be cooled more efficiently.

At least a part of the surface of the power supply line may be covered with an insulating material. The thickness of the insulating material covering the power supply line may be smaller than the thickness of the power supply line without being covered with the insulating material. In this case, as compared with a case where the thickness of the insulating material is equal to or greater than the thickness of the power supply line, heat propagated from the laser light source to the power supply line is more likely to be further released to the outside through the insulating material. Therefore, the laser light source can be cooled more efficiently.

The thickness of the insulating material covering the power supply line may, more preferably, be 25% or less of the thickness of the power supply line, and still more preferably 10% or less. In this case, heat propagated from the laser light source to the power supply line is more likely to be further released to the outside.

The light irradiation device may further include a supply detection unit configured to detect whether cooling liquid is being supplied into the cooling liquid passage, and a supply notification unit configured to notify a detection result by the supply detection unit. In this case, the user can appropriately ascertain whether or not the cooling liquid is being supplied into the cooling liquid passage. Accordingly, various problems resulting from laser light being emitted while the cooling liquid is not supplied are less likely to occur.

The specific configuration of the supply detection unit can be selected as appropriate. For example, the light irradiation device may include a passage valve that prevents backflow of the cooling liquid toward the proximal side in the cooling liquid passage (details will be described later). The supply detection unit may detect whether or not cooling liquid is being supplied into the cooling liquid passage by detecting whether the passage valve is open. In this case, whether or not the cooling liquid is supplied is appropriately detected based on whether or not the passage valve is open. The supply detection unit may also be a flow rate sensor or the like that is provided in at least a portion of the passage of the cooling liquid and detects the flow of the cooling liquid. It is also possible to use a temperature sensor as the supply detection unit. In this case, the temperature sensor may be used for both detection of the ambient temperature and detection of whether or not the cooling liquid is supplied.

The light irradiation device may further include a temperature detection unit configured to detect the temperature of the laser light source (or in the vicinity of the laser light source), and a temperature notification unit configured to notify a detection result by the temperature detection unit. In this case, the user can easily ascertain whether or not the temperature of the laser light source is appropriately maintained.

The specific configuration of the temperature detection unit can be selected as appropriate. For example, the temperature detection unit may be disposed on the laser light source. The temperature detection unit may detect the temperature in the vicinity of the laser light source (for example, the temperature of cooling water or the like in the vicinity of the laser light source). The control unit may detect whether or not the temperature of the laser light source is at or below a threshold by monitoring the state of the laser light source.

The light irradiation device may further include a passage valve. The passage valve is provided in the cooling liquid passage on the proximal side of the laser light source, and prevents backflow of the cooling liquid toward the proximal side. The passage valve prevents backflow of the cooling liquid in the cooling liquid passage. As a result, a rise in temperature of the laser light source and the vicinity thereof is more easily and appropriately suppressed.

The light irradiation system of the present disclosure is a light irradiation system for medical use including a catheter formed in a long tubular shape, and an elongated light irradiation device inserted into a lumen of the catheter. The light irradiation device includes a laser light source and a Peltier element. The laser light source is provided at a distal end portion of an elongated device body, and emits laser light. The Peltier element is disposed in contact with or in proximity to the laser light source has a cooling surface facing the laser light source. A light-transmitting portion that transmits, to an outside of the catheter, the laser light emitted by the laser light source in the light irradiation device is formed in at least a part of the distal end portion of the catheter.

According to the light irradiation system of the present disclosure, a problem caused by a rise in temperature of the distal end portion due to the laser light source is appropriately suppressed by a cooling effect of the Peltier element. Furthermore, the light emitted from the laser light source is transmitted through the light-transmitting portion of the catheter and is irradiated onto biological tissue. Therefore, light is irradiated more efficiently and appropriately to a specific position within a lumen of a living body. As described above, the cooling surface of the Peltier element and the laser light source may be spaced apart from each other to such an extent that a cooling effect of the Peltier element on the laser light source can be appropriately obtained. Further, the cooling surface of the Peltier element and the laser light source may be in proximity to each other in a state in which at least one of an adhesive and a member having high thermal conductivity is disposed between the cooling surface of the Peltier element and the laser light source.

Of the Peltier element, at least one of a heating surface located on a side opposite to the cooling surface and an indirect heat-dissipating member that is in contact with the heating surface and has a thermal conductivity equal to or higher than that of the heating surface may be externally exposed to a liquid (for example, a cooling liquid or the like). In this case, compared with a case where the heating surface is not directly or indirectly exposed, heat emitted from the heating surface of the Peltier element is more easily and appropriately dissipated.

In a state in which the light irradiation device is inserted into the catheter, a cooling liquid may be introduced into the lumen of the catheter. In this case, the heating surface of the Peltier element, or the vicinity of the heating surface, is more likely to come into contact with the cooling liquid introduced into the lumen of the catheter. As a result, the heat emitted from the heating surface of the Peltier element can be dissipated more easily and efficiently.

### [Brief Description of Drawings]

[FIG. 1] FIG. 1 is a longitudinal sectional view of a light irradiation system 1 in a state in which a light irradiation device 2 and a catheter 3 are separated from each other.
[FIG. 2] FIG. 2 is a longitudinal sectional view of the light irradiation system 1 in a state in which the light irradiation device 2 is attached to the catheter 3 (a use state).
[FIG. 3] FIG. 3 is an enlarged longitudinal sectional view of the vicinity of the distal end portion of the light irradiation system 1 in FIG. 2.
[FIG. 4] FIG. 4 is a cross-sectional view of the power supply line 231 taken in a direction perpendicular to the axis O2 of the light irradiation device 2.
[FIG. 5] FIG. 5 is an enlarged longitudinal sectional view of the vicinity of the distal end portion of the light irradiation system 1 according to a first modified example.
[FIG. 6] FIG. 6 is an enlarged longitudinal sectional view of the vicinity of the distal end portion of the light irradiation system 1 according to a second modified example.
[FIG. 7] FIG. 7 is an enlarged longitudinal sectional view of the vicinity of the distal end portion of the light irradiation system 1 according to a third modified example.

### [Detailed Description of Embodiments]

Hereinafter, exemplary embodiments of the present disclosure will be described with reference to the drawings. The light irradiation system 1 of the present embodiment is used by being inserted into a body lumen of a living body (for example, at least one of a blood vessel, a lymph gland, a urethra, an airway, a digestive organ, a secretory gland, and a reproductive organ). The light irradiation system 1 irradiates biological tissue with light (laser light in the present embodiment) while being inserted into the body lumen of the living body. The light irradiation system can be used for at least one of therapies such as PDT (Photodynamic Therapy) and NIR-PIT (Near-infrared photoimmunotherapy).

The light irradiation system 1 of the present embodiment includes a light irradiation device 2 and a catheter 3. When the light irradiation system 1 is used, first, the catheter 3 is inserted into a body lumen. Next, the light irradiation device 2 is inserted into the lumen 311 of the catheter 3, which has an elongated tubular shape. Upon completion of the insertion, light is emitted from the light irradiation device 2 to biological tissue. However, it is also possible to use only the light irradiation device 2 alone without using the catheter 3.

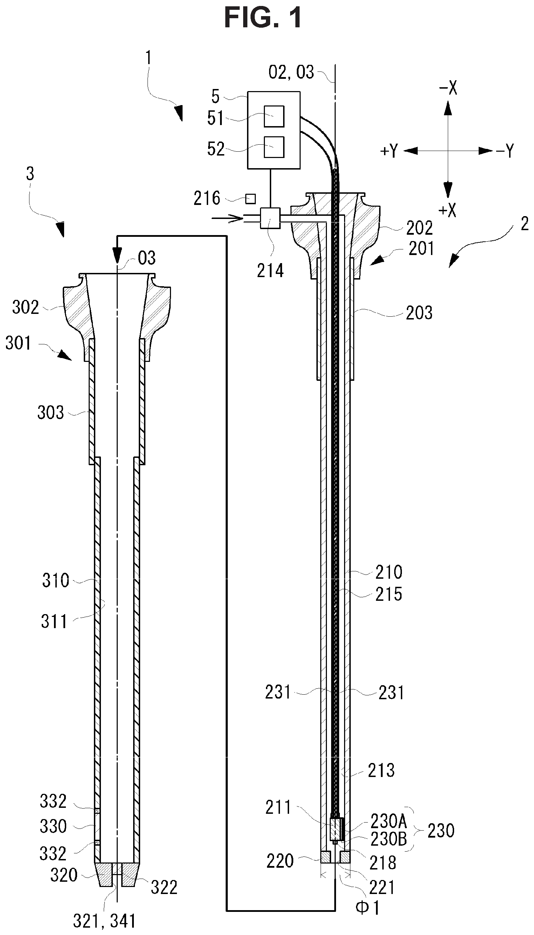

FIGS. 1 to 3 and 5 to 7 illustrate XY axes orthogonal to each other. In these drawings, the lower side of the drawings (+X direction) is defined as the "distal side," the upper side of the drawings (-X direction) is defined as the "proximal side," the left side of the drawings (+Y direction) is defined as the "left side," and the right side of the drawings (-Y direction) is defined as the "right side." The light irradiation system 1, the light irradiation device 2, and the catheter 3 are inserted into the body lumen from the distal side. The proximal side is operated by a medical professional (for example, a doctor or the like).

### (Light Irradiation Device)

With reference to FIGS. 1 to 4, the light irradiation device 2 according to the present embodiment will be described. As shown in FIG. 1, the light irradiation device 2 is elongated in shape. The light irradiation device 2 includes a connector 201, a device body 210, a laser light source 211, and a distal tip 220. The connector 201 is located on the proximal side of the light irradiation device 2 and is gripped by an operator. The connector 201 includes a pair of wing portions 202 and a connecting portion 203. The connecting portion 203 is a substantially cylindrical member. The wing portions 202 are connected to the proximal end portion of the connecting portion 203. The device body 210 is connected to the distal end portion of the connecting portion 203. Note that the wing portions 202 and the connecting portion 203 may be integrally formed. The device body 210 is an elongated member extending along the axis O2. The laser light source 211 is a compact laser light source that emits laser light in a predetermined wavelength range. The laser light source 211 is provided at the distal end portion of the elongated device body 210. As one example, in the present embodiment, the laser light source 211 is installed at the distal end of an elongated torque coil 215 provided inside the device body 210. However, the specific method for installing the laser light source 211 may also be changed. For example, a member other than the torque coil 215 may be used. The laser light source 211 may be directly fixed to the distal end portion of the device body 210. The distal tip 220 is connected to the distal end portion of the device body 210 at a position further on the distal side than the laser light source 211. The outer diameter of the distal tip 220 is substantially the same as the outer diameter Φ1 of the device body 210.

It is desirable that the device body 210 have antithrombogenicity, flexibility, and biocompatibility. As the material of the device body 210, at least one of a resin material and a metal material can be employed. Examples of the resin material include polyamide resin, polyolefin resin, polyester resin, polyurethane resin, silicone resin, and fluororesin. The device body 210 of the present embodiment is formed of a resin material that transmits laser light emitted from the laser light source 211 described below. Therefore, it is not necessary to separately form, in the device body 210, a transmissive portion that transmits the laser light. However, when a transmissive portion that transmits laser light emitted from the laser light source 211 is formed in the device body 210, or when the laser light source 211 is exposed outside the device body 210, the device body 210 may be formed of a material that does not transmit laser light (for example, a metal material or the like). Examples of the metal material include stainless steel such as SUS304, nickel-titanium alloy, cobalt-chromium alloy, platinum, and tungsten steel. It is also possible to configure the device body 210 by combining a plurality of materials.

The device body 210 includes a cooling liquid passage 213. The cooling liquid passage 213 extends from the proximal side (the -X side in the drawings) of the device body 210 to the laser light source side (the +X side in the drawings), and allows a cooling liquid for cooling the laser light source 211 to pass to the laser light source 211 side (that is, to the distal side of the device body 210). Accordingly, in the light irradiation device 2 of the present embodiment, a rise in temperature of the laser light source 211 and the vicinity thereof is appropriately suppressed by the cooling liquid supplied to the laser light source 211 through the cooling liquid passage 213. Therefore, problems caused by a rise in temperature of the distal end portion due to the laser light source 211 (for example, at least one of failure of the laser light source 211 and coagulation of blood) are appropriately suppressed. In the present embodiment, the device body 210 is formed in a long tubular shape, whereby the cooling liquid passage 213 is defined within the inner cavity (lumen) of the device body 210. The cooling liquid passage 213 of the present embodiment extends to a position further on the distal side than the laser light source 211. However, the cooling liquid passage 213 only needs to extend toward the laser light source 211 to such an extent that it can supply the cooling liquid to the laser light source 211. Accordingly, the distal end portion of the cooling liquid passage 213 may be located on the proximal side of the laser light source 211.

As shown in FIG. 1, the cooling liquid is supplied into the cooling liquid passage 213 from the proximal side thereof. The light irradiation device 2 includes a passage valve 214. The passage valve 214 is provided on the proximal side of the laser light source 211 in the cooling liquid passage 213. The passage valve 214 prevents the cooling liquid from flowing back toward the proximal side in the cooling liquid passage 213, and allows the cooling liquid to flow toward the distal side. As a result, a problem in which the cooling liquid is not supplied to the laser light source 211 and the vicinity thereof is suppressed, thereby facilitating a further appropriate suppression of a rise in temperature of the laser light source 211 and the vicinity thereof. Note that, as the cooling liquid, various liquids that do not affect biological tissue (for example, physiological saline or the like) can be used.

At the distal end portion of the device body 210, a discharge port 221 is formed for discharging, to the outside of the device body 210, the cooling liquid supplied into the interior from the proximal side of the cooling liquid passage 213. Accordingly, the cooling liquid supplied into the cooling liquid passage 213 smoothly flows in the vicinity of the laser light source 211 without stagnating therein. As a result, a rise in temperature of the laser light source 211 and the vicinity thereof is further appropriately suppressed more easily.

In the present embodiment, the discharge port 221 is formed in the distal tip 220 provided at the distal end portion of the device body 210. However, the specific configuration of the discharge port may be changed. For example, separately from the discharge port 221 of the distal tip 220, or together with the discharge port 221 of the distal tip 220, a discharge port may be formed in a side surface of the device body 210 having an elongated tubular shape. Preferably, the discharge port is formed on a more distal side than the position where the laser light source 211 is installed, in the extending direction of the device body 210 (the direction of the axis O2).

Further, in the present embodiment, at least a part of the distal tip 220 provided at the distal end portion of the light irradiation device 2 (in the present embodiment, the entirety of the distal tip 220) is formed of a radiopaque material. Accordingly, when causing the light irradiation device 2 to irradiate a biological tissue with laser light while imaging the inside of a living body using radiation (for example, X-rays or the like), medical personnel (for example, an operator or the like) can appropriately adjust the irradiation position of the laser light by checking the position of the distal tip 220 appearing in the captured image. Therefore, treatment accuracy is likely to be improved.

The light irradiation device 2 includes a supply detection unit 216 and a supply notification unit 51. The supply detection unit 216 detects whether or not the cooling liquid is supplied into the cooling liquid passage 213. The supply notification unit 51 notifies the user of the detection result by the supply detection unit 216. Accordingly, the user can appropriately ascertain whether or not the cooling liquid is supplied into the cooling liquid passage 213. Therefore, various malfunctions caused by emission of the laser light without the cooling liquid being supplied are less likely to occur.

Specific configurations of the supply detection unit 216 and the supply notification unit 51 may be appropriately selected. As an example, the supply detection unit 216 of the present embodiment detects whether or not the cooling liquid is supplied into the cooling liquid passage 213 by detecting whether or not the passage valve 214 is open. Accordingly, the presence or absence of supply of the cooling liquid is appropriately detected based on whether or not the passage valve 214 is open. Further, the control unit 5 notifies the user of the detection result by controlling driving of the supply notification unit 51 (for example, driving of at least one of turning on, turning off, and blinking of a light source, audio output, and image display) according to the detection result by the supply detection unit 216. However, the configurations of the supply detection unit 216 and the supply notification unit 51 may be changed. For example, the supply detection unit may be a flow rate sensor or the like that detects the flow of the cooling liquid by being provided in at least a part of the passage of the cooling liquid. Further, a temperature sensor may be used as the supply detection unit. In this case, the temperature sensor may be used for both detection of the ambient temperature and detection of the presence or absence of supply of the cooling liquid.

The light irradiation device 2 includes a temperature detection unit 218 and a temperature notification unit 52. The temperature detection unit 218 detects the temperature of at least one of the laser light source 211 and the vicinity of the laser light source 211. The temperature notification unit 52 notifies the user of the detection result by the temperature detection unit 218. Accordingly, the user can easily ascertain whether or not the temperature of the laser light source 211 (or the vicinity thereof) is appropriately maintained.

The specific configurations of the temperature detection unit 218 and the temperature notification unit 52 can be selected as appropriate. As one example, the temperature detection unit 218 of the present embodiment is provided on the laser light source 211 and thereby detects the temperature of the laser light source 211. However, the temperature detection unit 218 may detect the temperature in the vicinity of the laser light source 211 (for example, the temperature of cooling liquid or the like in the vicinity of the laser light source 211). Further, the control unit 5 may detect whether or not the temperature of the laser light source 211 is equal to or lower than a threshold value by monitoring the state of the laser light source 211. Further, in the present embodiment, the control unit 5 notifies the user of the detection result by controlling driving of the temperature notification unit 52 (for example, driving of at least one of turning on, turning off, and blinking a light source, audio output, and image display) in accordance with the detection result by the temperature detection unit 218. The control unit 5 may notify whether or not the temperature detected by the temperature detection unit 218 is equal to or lower than a threshold value, or may notify the detected temperature itself.

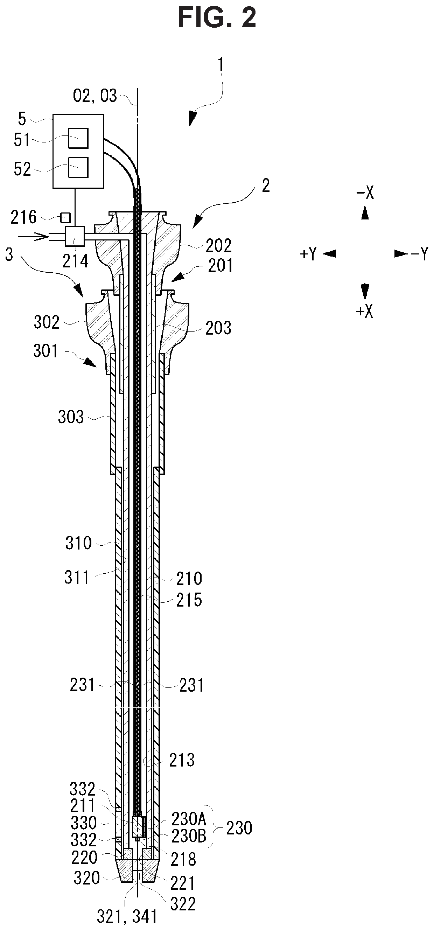

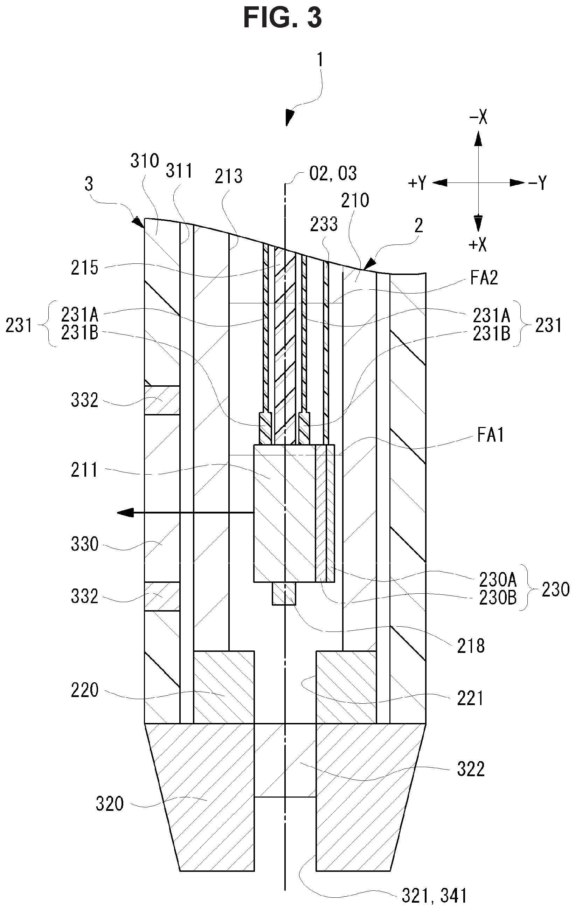

With reference to FIG. 3, the configuration of the distal end portion of the light irradiation device 2 according to the present embodiment will be described. FIG. 3 is an enlarged longitudinal sectional view of the vicinity of the distal end portion of the light irradiation system 1 in FIG. 2. As described above, the light irradiation device 2 includes, at the distal end portion thereof, a small-sized laser light source 211 that emits laser light in a predetermined wavelength range. More specifically, the laser light source 211 is formed in a rectangular shape, and the laser light source 211 is disposed in the cooling liquid passage 213, which is an inner cavity (lumen) of the light irradiation device 2 having an elongated tubular shape. However, the method for fixing the laser light source 211 can also be changed. For example, at least a part of the laser light source 211 (for example, a light-emitting unit that emits laser light) may be exposed outside the device body 210.

The laser light source 211 emits laser light in a direction intersecting the longitudinal direction of the light irradiation device 2 (the direction of the axis O2) (in the example shown in FIG. 3, the direction of the arrow intersecting the axis O2 perpendicularly). Accordingly, the light irradiation device 2 can directly irradiate a specific position of a living body with light from the laser light source 211 provided at the distal end portion thereof, without using an optical transmission member such as an optical fiber. Therefore, occurrence of various problems that arise when an optical transmission member is used (for example, at least one of a problem in which light leakage or attenuation occurs in the middle of the optical transmission member, and a problem in which characteristics of the light change in the course of transmission) is appropriately suppressed. Further, the laser light source 211 emits laser light from the distal end portion of the light irradiation device 2 in a direction intersecting the direction of the axis O2. Compared with a light-emitting diode, the laser light source 211 can easily emit light that is less likely to diverge and has higher directivity. Accordingly, the light irradiation device 2 of the present embodiment can selectively irradiate a specific position of a living body with the laser light emitted from the laser light source 211. As a result, various problems caused by light being irradiated to an unintended position (for example, occurrence of side effects and the like) are also less likely to occur. Furthermore, the laser light source 211 has a property of being capable of irradiating light having a narrower spectral width than a light-emitting diode. Accordingly, by providing the laser light source 211 at the distal end portion of the light irradiation device 2, occurrence of various problems (for example, at least one of a decrease in irradiation efficiency and an unintended change in tissue) due to tissue being irradiated with a wavelength different from the wavelength required for treatment (for example, an excitation wavelength of a photosensitive substance) is also suppressed. Therefore, it becomes easier to irradiate a specific position in a lumen of a living body with light more efficiently and appropriately.

The laser light source 211 may employ a surface-emitting laser that emits laser light in a direction perpendicular to a substrate. By using the surface-emitting laser, the light irradiation device 2 appropriately emits laser light with low power and also has high resistance to temperature changes. Furthermore, since the surface-emitting laser can emit laser light in a direction perpendicular to the substrate surface, it becomes easier to more accurately adjust the irradiation position of the laser light.

The laser light source 211 may also employ a semiconductor laser, which is a circuit element manufactured using a semiconductor material. Since the semiconductor laser can be easily miniaturized, it can also be easily and appropriately incorporated into the light irradiation device 2 having a small diameter. In addition, the semiconductor laser can emit highly directional laser light having aligned phases with low power. Therefore, the therapeutic effect also tends to be stable.

The laser light source 211 may emit laser light having a wavelength of 300 nm or more and 2000 nm or less. More preferably, the laser light source 211 may emit laser light having a wavelength of 600 nm or more and 1000 nm or less. In this case, by using the light irradiation device 2 for treatment of a disease using a photosensitive substance, the therapeutic effect can be more readily and appropriately obtained. In the present embodiment, the center wavelength of the laser light emitted from the laser light source 211 is set to about 690 nm.

The light irradiation device 2 includes power supply lines 231 (in the present embodiment, a pair of power supply lines 231). The power supply lines 231 extend from the proximal end side to the distal end side of the device body 210, and, by being connected to the laser light source 211, supply at least electrical power to the laser light source 211. In the present embodiment, the proximal end side of the power supply lines 231 is connected to the control unit 5 (see FIGS. 1 and 2). The power supply lines 231 (in the present embodiment, both of the pair of power supply lines 231) are exposed within the cooling liquid passage 213 in the device body 210. Therefore, heat generated from the laser light source 211 is more readily released to the cooling liquid through the power supply lines 231. As a result, the laser light source 211 can be cooled more efficiently. In the present embodiment, as the material of the power supply lines 231, a metal material having high thermal conductivity (for example, at least one of copper and nickel) is employed. Therefore, heat generated from the laser light source 211 is more readily conducted to the power supply lines 231. The heat conducted to the power supply lines 231 is smoothly released to the cooling liquid.

As shown in FIG. 3, in the power supply lines 231 (in the present embodiment, each of the pair of power supply lines 231), an outer diameter of a connecting portion 231B connected to the laser light source 211 is larger than an outer diameter of a proximal end portion 231A extending from the connecting portion 231B toward the proximal side (+X side in FIG. 3). In other words, when the cross-sectional area of the power supply line 231 in a cross section orthogonal to the axis O2 of the light irradiation device 2 is considered, the cross-sectional area of the connecting portion 231B connected to the laser light source 211 is larger than the cross-sectional area of the proximal end portion 231A extending from the connecting portion 231B toward the proximal side. Therefore, compared with, for example, a case in which the cross-sectional area of the power supply line 231 is constant, heat generated from the laser light source 211 is more readily conducted to the power supply line 231. The heat conducted to the power supply line 231 is smoothly released to the cooling liquid. Accordingly, the laser light source 211 can be cooled more efficiently.

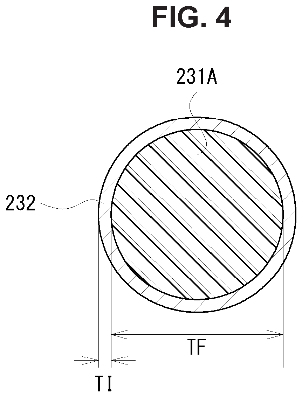

FIG. 4 is a cross-sectional view of the power supply line 231 in a direction perpendicular to the axis O of the light irradiation device 2. As shown in FIG. 4, at least a part of the power supply line 231 of the present embodiment (in the present embodiment, both the proximal end portion 231A and the connecting portion 231B of the power supply line 231) is covered with an insulating material 232. As a result, electrical leakage from the power supply line 231 to the outside is appropriately suppressed. As the material of the insulating material 232, for example, at least one of materials having insulating properties, such as polyurethane, polyester, polyesterimide, polyamideimide, and polyimide, may be employed.

In the present embodiment, the shape of a cross-section, perpendicular to the axis O2, of the power supply line 231, which is elongated, is circular. However, the cross-sectional shape of the power supply line 231 may be a shape other than a circular shape (for example, a rectangular shape or an elliptical shape).

As shown in FIG. 4, in the present embodiment, the thickness TI of the insulating material 232 covering the power supply line 231 is designed to be smaller than the thickness TF of the power supply line 231 in a state in which the power supply line 231 is not covered with the insulating material 232 (specifically, the thickness of the proximal-side portion 231A of the power supply line 231). The term "thickness" means a thickness in a cross-section perpendicular to the extending direction of the power supply line 231. In other words, in the present embodiment, the thickness TI of the insulating material 232 is designed to be smaller than the diameter TF of the power supply line 231. As a result, since the thickness TI of the insulating material 232 is reduced, heat propagated from the laser light source 211 to the power supply line 231 is more likely to be released to the outside (the cooling liquid in the present embodiment) through the thinly formed insulating material 232. Accordingly, the laser light source 211 can be cooled more efficiently. As one example, the thickness of the power supply line 231 may be designed to be 0.05 mm to 0.20 mm, and the thickness of the insulating material 232 may be designed to be 0.003 mm to 0.02 mm. It should be noted that the thickness of the insulating material 232 covering the power supply line 231 may more preferably be 25% or less of the thickness of the power supply line 231, and still more preferably 10% or less. In this case, heat propagated from the laser light source to the power supply line is even more likely to be released to the outside.

As shown in FIG. 3, in the present embodiment, the device body 210 is formed in an elongated tubular shape, whereby a cooling liquid passage 213 is provided in a lumen of the device body 210. Here, an area of the cooling liquid passage 213 in a cross-section in a direction intersecting perpendicularly with the extending direction of the device body 210 (the direction of the axis O2) is defined as a passage area. In the present embodiment, in the extending direction of the device body 210 (the direction of the axis O2), a passage area FA2 of the cooling liquid passage 213 at a portion located on a proximal side of the laser light source 211 is larger than a passage area FA1 of the cooling liquid passage 213 at a portion where the laser light source 211 is disposed. As a result, pressure loss of the cooling liquid is less likely to occur in a path within the device body 210 up to the vicinity of the laser light source 211, and the cooling liquid is more likely to appropriately flow in the vicinity of the laser light source 211. Accordingly, the laser light source can be cooled more efficiently.

A range in which the passage area is made larger than at the portion where the laser light source 211 is disposed (that is, a range of the "portion on the proximal side of the laser light source") can be appropriately set. As one example, in the present embodiment, the passage area over an entire range extending toward the proximal side from the proximal end of the laser light source 211 itself is larger than the passage area at the portion where the laser light source 211 is disposed. However, a certain distance (for example, when a length of the laser light source 211 in the direction of the axis O2 is L, a distance equal to or less than the length L) may be provided between the range in which the passage area is made larger than at the portion where the laser light source 211 is disposed and the proximal end of the laser light source 211 itself.

In the present embodiment, as shown in FIG. 3, while the inner diameter of the cooling liquid passage 213 is kept constant, a cross-sectional area of a member adjacent on the proximal side of the laser light source 211 is made smaller than a cross-sectional area of a member at a portion where the laser light source 211 is located (in the present embodiment, a sum of the cross-sectional area of the laser light source 211 and the cross-sectional area of a Peltier element 230 described later). In this case, with the shape of the device body 210 forming the cooling liquid passage 213 simplified, the passage area FA1 of the portion where the laser light source 211 is disposed can be made smaller than the passage area FA2 of an adjacent portion on the proximal side of the laser light source 211. However, it is also possible to change the method of making the passage area FA2 larger than the passage area FA1. For example, the inner diameter of the cooling liquid passage 213 at the portion where the laser light source 211 is disposed may be made smaller than the inner diameter of the cooling liquid passage 213 at an adjacent portion on the proximal side of the laser light source 211. In this case, while reducing the diameter of the light irradiation device 2 in the vicinity of the laser light source 211, the passage area FA1 of the portion where the laser light source 211 is disposed can be made smaller than the passage area FA2 of an adjacent portion on the proximal side of the laser light source 211.

As shown in FIG. 3, the light irradiation device 2 of the present embodiment includes the Peltier element 230 at a distal end portion of the device body 210. Wiring 233 extending from the proximal side along an extending direction of the light irradiation device 2 (a direction of the axis O2) is connected to the Peltier element 230 (illustration of the wiring 233 is omitted in FIGS. 1 and 2). The Peltier element 230 is a semiconductor element using the Peltier effect, and has a heating surface 230A and a cooling surface 230B. When a direct current is passed through the Peltier element 230, the cooling surface 230B absorbs heat, while the heating surface 230A generates heat. The Peltier element 230 is disposed in contact with the laser light source 211 with the cooling surface 230B facing the laser light source 211. However, the cooling surface 230B of the Peltier element 230 may be disposed at a position close to the laser light source 211 (that is, at a position where a gap is formed therebetween) with the cooling surface 230B facing the laser light source 211. In other words, the cooling surface 230B of the Peltier element 230 and the laser light source 211 may be spaced apart from each other to an extent that the cooling effect of the Peltier element 230 on the laser light source 211 is appropriately obtained. Further, the cooling surface 230B of the Peltier element 230 and the laser light source 211 may be close to each other with at least one of an adhesive and a member having high thermal conductivity or the like disposed between the cooling surface 230B of the Peltier element 230 and the laser light source 211. By causing the cooling surface 230B side of the Peltier element 230 to face the laser light source 211, various problems caused by a temperature rise of the distal end portion due to the laser light source 211 are appropriately suppressed by the cooling effect of the Peltier element 230.

In the present embodiment, an outer periphery of the Peltier element 230 is covered with a covering member (for example, resin or the like) that prevents liquid from entering its interior. As a result, malfunctions such as failure of the Peltier element 230 due to ingress of liquid are appropriately suppressed.

The heating surface 230A of the Peltier element 230 is exposed externally so as to be exposed to liquid. Specifically, in the present embodiment, the heating surface 230A is exposed in a space within the cooling liquid passage 213 through which cooling liquid flows. Accordingly, when cooling liquid is supplied into the cooling liquid passage 213, the heating surface 230A of the Peltier element 230 is exposed to (comes into contact with) the cooling liquid. Therefore, compared with a case where the heating surface 230A is not exposed externally, heat emitted from the heating surface 230A of the Peltier element 230 can be more easily and appropriately released to the outside.

In the present embodiment, at least a portion of the Peltier element 230 (for example, at least one of the heating surface 230A and the cooling surface 230B) is formed of a radiopaque material. Accordingly, when medical personnel cause the light irradiation device 2 to irradiate biological tissue with laser light while imaging the inside of a living body using radiation, the irradiation position of the laser light can be appropriately adjusted by confirming the position of the Peltier element 230 appearing in the captured image. Specifically, in the present embodiment, the installation position of the Peltier element 230 in the light irradiation device 2 is offset from the axis O2 of the light irradiation device 2. Accordingly, medical personnel can appropriately ascertain the emission direction of the laser light from the laser light source 211 by confirming, in the captured image, the position of the Peltier element 230 relative to the axis O2 of the light irradiation device 2.

### (Catheter)

The catheter 3 according to the present embodiment will be described with reference to FIGS. 1 to 3. As shown in FIG. 1, the catheter 3 has an elongated tubular shape. The catheter 3 includes a connector 301, a shaft 310, and a distal tip 320. The connector 301 is located on the proximal end of the catheter 3 and is gripped by an operator. The connector 301 includes a pair of wing portions 302 and a connecting portion 303. The connecting portion 303 is a substantially cylindrical member. The wing portions 302 are connected to a proximal end portion of the connecting portion 303. The shaft 310 is connected to a distal end portion of the connecting portion 303. Note that the wing portions 302 and the connecting portion 303 may be integrally formed.

Similarly to the device body 210 of the light irradiation device 2, the shaft 310 preferably has antithrombogenicity, flexibility, and biocompatibility. As the material of the shaft 310, a material similar to that of the device body 210 of the light irradiation device 2 can be employed. The shaft 310 is an elongated tubular member extending along an axis O3. In the present embodiment, the shaft 310 is formed in a hollow cylindrical shape with both a distal end portion and a proximal end portion open. During delivery of the catheter 3, a lumen 311 inside the shaft 310 functions as a guidewire lumen for passing a guidewire through the catheter 3. After delivery of the catheter 3, the lumen 311 functions as a device lumen for passing the light irradiation device 2 through the catheter 3.

The distal tip 320 is connected to a distal end portion of the shaft 310. The distal tip 320 has an outer shape tapered in diameter from the proximal side toward the distal side so that the catheter 3 can smoothly advance within a body lumen. A through-hole 321 penetrating in the direction of the axis O2 is formed at a substantially central portion of the distal tip 320. The inner diameter of the through-hole 321 is smaller than the inner diameter of the lumen 311 of the shaft 310, and is also smaller than the outer diameter Φ1 of the distal tip 220 of the light irradiation device 2. Further, the outer diameter Φ1 of each of the device body 210 and the distal tip 220 of the light irradiation device 2 is equal to or smaller than the inner diameter of the lumen 311 of the catheter 3. Accordingly, the light irradiation device 2 moves within the lumen 311 of the catheter 3 along the axis O2. When the light irradiation device 2 is sufficiently pushed forward within the lumen 311 of the catheter 3, the distal tip 220 of the light irradiation device 2 comes into contact with the distal tip 320 of the catheter 3, thereby positioning the light irradiation device 2 relative to the catheter 3 in the directions of the axes O2 and O3. Note that at least a part of the distal tip 320 (in the present embodiment, the entirety of the distal tip 320) is formed of a material having radiopacity. Accordingly, the position of the distal end portion of the catheter 3 can be appropriately ascertained by an image obtained using radiation.

In the present embodiment, a cooling liquid is supplied into the lumen 311 of the catheter 3 (the space between the outer peripheral surface of the light irradiation device 2 and the inner peripheral surface of the lumen 311 of the catheter 3). That is, in the present embodiment, the cooling liquid is supplied to both the cooling liquid passage 213 of the light irradiation device 2 and the lumen 311 of the catheter 3. As a result, problems caused by a temperature rise at the distal end portion due to the laser light source 211 can be more readily suppressed.

As shown in FIG. 3, the catheter 3 is provided, on a distal-side lateral surface of the shaft 310 (in the present embodiment, a part of the distal-side lateral surface), with a light-transmitting portion 330 that transmits, to the outside of the catheter 3, laser light emitted by the laser light source 211 in the light irradiation device 2. Accordingly, the light irradiation system 1 according to the present embodiment is capable of selectively irradiating a specific position of a living body with laser light emitted, by the laser light source 211 of the light irradiation device 2, in a direction intersecting the axes O2 and O3.

In the present embodiment, the light-transmitting portion 330 is provided by partially forming, with a material that transmits laser light, a portion of the shaft 310 of the catheter 3 through which the laser light emitted by the laser light source 211 is transmitted. However, the configuration of the light-transmitting portion may also be changed. For example, the catheter may be provided with a light-transmitting portion by using, as the material of the shaft 310 itself or of the entirety of the distal end portion of the shaft 310, a material that transmits laser light.

The shaft 310 of the catheter 3 is provided, at a position adjacent to the light-transmitting portion 330, with a catheter-side marker portion 332 having radiopacity. Accordingly, when causing a biological tissue to be irradiated with laser light by the light irradiation device 2 while imaging the inside of a living body using radiation, a medical practitioner can appropriately irradiate the laser light from the light-transmitting portion 330 to the outside by aligning the position of the laser light source 211 of the light irradiation device 2 with the position of the catheter-side marker portion 332 appearing in the captured image. Therefore, the accuracy of treatment can be more readily improved.

As shown in FIG. 3, a discharge port 341 for discharging the cooling liquid from the inside of the lumen 311 to the outside of the catheter 3 is formed in the distal end portion of the catheter 3. Accordingly, the cooling liquid supplied into the catheter 3 (in the present embodiment, both the cooling liquid supplied to the cooling liquid passage 213 of the light irradiation device 2 and the cooling liquid supplied into the lumen 311 of the catheter 3) passes through the vicinity of the distal end portion of the light irradiation device 2 where the laser light source 211 is installed, and is discharged from the discharge port 341 to the outside of the catheter 3. As a result, since the cooling liquid continues to be supplied to the vicinity of the laser light source 211, a temperature rise in the laser light source 211 and its vicinity can be more appropriately suppressed. Further, the possibility that blood outside the catheter 3 may come into contact with members inside the catheter 3 (for example, the laser light source 211 and the like) is also appropriately reduced. Therefore, coagulation of blood and the like due to heat from the laser light source 211 is also less likely to occur.

In the catheter 3 of the present embodiment, a through hole 321 of the distal tip 320 for inserting a guide wire during delivery of the catheter 3 also serves as the discharge port 341 for the cooling liquid. Accordingly, while the configuration of the catheter 3 is prevented from becoming complicated, both delivery of the catheter 3 and cooling in the vicinity of the laser light source 211 are appropriately performed. However, the specific configuration of the discharge port of the catheter 3 can also be changed. For example, separately from the through hole 321 of the distal tip 320, or together with the through hole 321 of the distal tip 320, a discharge port may be formed in a side surface of the shaft 310 having an elongated tubular shape. It is preferable that the discharge port be formed, in the extending direction of the catheter 3 (the direction of the axis O3), further toward the distal end side than the position at which the laser light source 211 is disposed during use.

As shown in FIG. 3, the discharge port 341 of the catheter 3 is provided with a discharge valve 322 that allows discharge of liquid to the outside of the catheter 3 through the discharge port 341 while preventing inflow of liquid from the outside to the inside of the catheter 3. As a result, the discharge valve 322 suppresses blood or the like outside the catheter 3 from flowing into the inside of the catheter 3 through the discharge port 341. Therefore, coagulation of blood or the like due to heat of the laser light source 211 is further less likely to occur.

### (Method of Use)

An example of a method of using the light irradiation system 1 of the present embodiment will be described. First, the operator inserts a guide wire (not shown) into a biological lumen. Next, the operator inserts a proximal end of the guide wire from the through hole 321 of the distal tip 320 of the catheter 3 into the lumen 311, and causes it to protrude from the proximal end of the connector 301. The operator advances the catheter 3 along the guide wire and moves the light-transmitting portion 330 of the catheter 3 to a target site for light irradiation. When moving the catheter 3 within the biological lumen, the operator can appropriately move the catheter 3 to the target site by confirming the position of the catheter-side marker portion 332 on a radiographic image. Thereafter, the operator withdraws the guide wire from the catheter 3.

The operator supplies a cooling liquid to the cooling liquid passage 213 of the light irradiation device 2 and to the lumen 311 of the catheter 3. The operator inserts the light irradiation device 2 through the proximal-side opening of the connector 301 of the catheter 3 and advances the light irradiation device 2 within the biological lumen along the lumen 311 of the catheter 3. When the light irradiation device 2 has been sufficiently advanced within the lumen 311 of the catheter 3, the distal tip 220 of the light irradiation device 2 comes into contact with the distal tip 320 of the catheter 3. As shown in FIG. 3, the light-transmitting portion 330 of the catheter 3 is formed at a position, in the axial direction of the axes O2 and O3, at which the laser light source 211 is disposed in a state where the distal tip 220 of the light irradiation device 2 is in contact with the distal end of the lumen of the catheter 3 (the distal tip 320 of the catheter 3). Therefore, merely by advancing the light irradiation device 2 until it comes into contact with the distal tip 320 of the catheter 3, the position of the laser light source 211 and the position of the light-transmitting portion 330 in the axial direction of the axes O2 and O3 automatically coincide with each other. Further, the operator adjusts the emission direction of the laser light from the laser light source 211 by rotating the light irradiation device 2 about the axis O2 while confirming, on a radiographic image, the position of the Peltier element 230 with respect to the axes O2 and O3. In this state, by emitting laser light from the laser light source 211, the target site is selectively irradiated with the laser light.

### (Modifications)

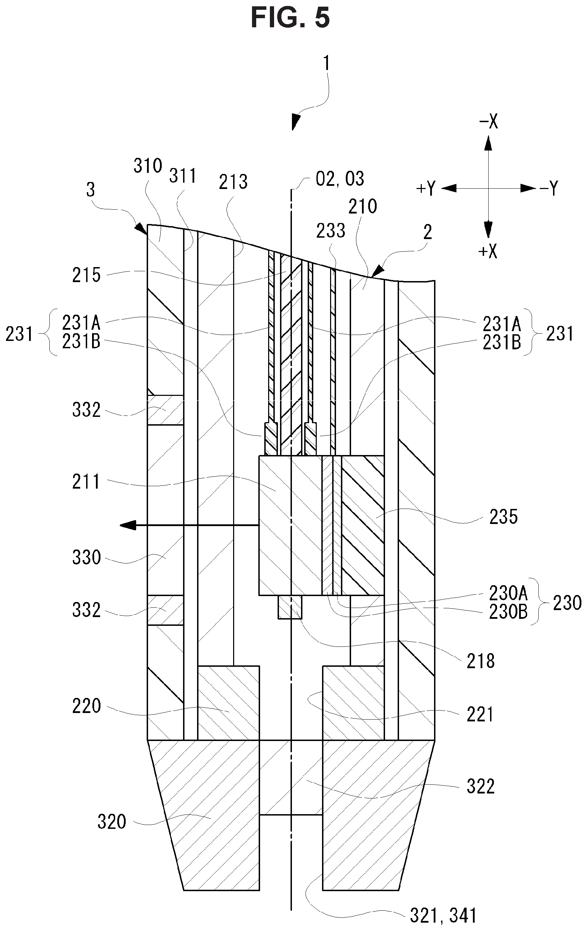

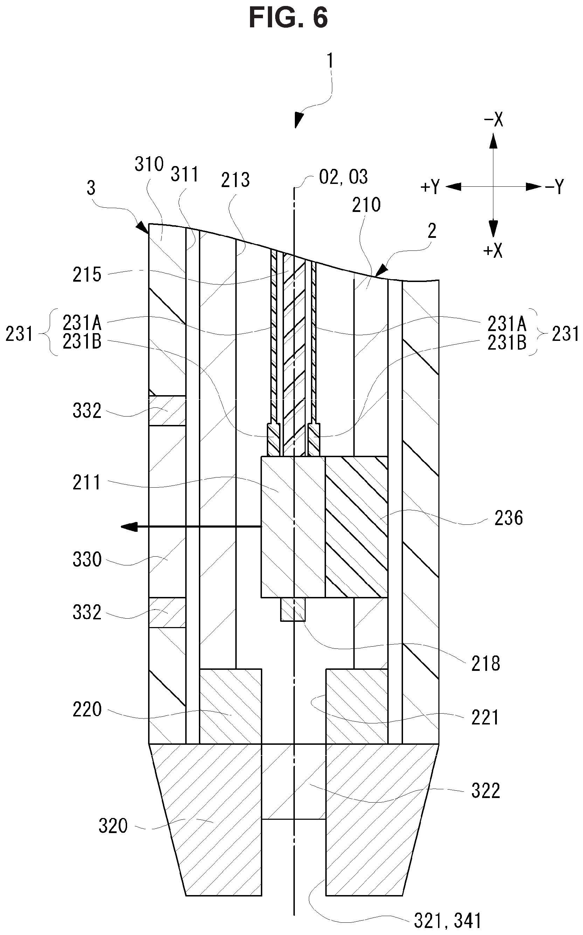

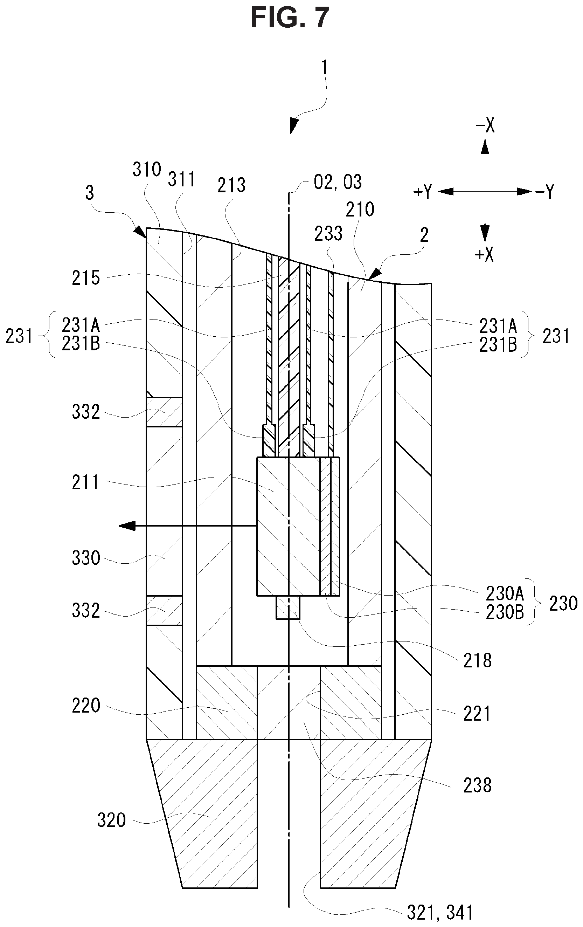

The technology disclosed in the above embodiment is merely an example. Therefore, the technology exemplified in the above embodiment can also be modified. With reference to FIGS. 5 to 7, some modifications of the above embodiment will be described. It should be noted that some configurations of the first modification shown in FIG. 5, the second modification shown in FIG. 6, and the third modification shown in FIG. 7 can employ configurations similar to those of the above-described embodiment. Accordingly, among the configurations of the first to third modifications, portions for which configurations similar to those of the above-described embodiment can be employed are denoted by the same reference numerals as those of the above embodiment, and descriptions thereof are omitted or simplified.

In the light irradiation system 1 according to the first modification shown in FIG. 5, an indirect heat-dissipating member 235 is arranged in contact with the heating surface 230A of the Peltier element 230. For the indirect heat-dissipating member 235, a member having a thermal conductivity equal to or greater than that of the heating surface 230A (for example, at least one of a heat pipe, carbon nanotubes, ceramics (AIN, SiC, etc.), a metal plate (platinum, titanium, copper, etc.), and a torque wire) is employed. When a metal plate is used, the metal plate may be an alloy, or the surface of the metal plate may be plated. The indirect heat-dissipating member 235 is externally exposed to a liquid. As one example, the indirect heat-dissipating member 235 of the light irradiation system 1 shown in FIG. 5 is exposed to the outside of the device body 210 from a side surface of the device body 210 of the light irradiation device 2. Therefore, the indirect heat-dissipating member 235 is exposed to the cooling liquid supplied into the lumen 311 of the catheter 3 (the space between the outer peripheral surface of the light irradiation device 2 and the inner peripheral surface of the lumen 311 of the catheter 3). Accordingly, the heat emitted from the heating surface 230A of the Peltier element 230 can be readily and appropriately released to the outside through the indirect heat-dissipating member 235.

In the light irradiation system 1 according to the second modification shown in FIG. 6, unlike the other embodiments, no Peltier element for cooling the laser light source 211 is used. However, in the light irradiation system 1 shown in FIG. 6, a heat-dissipating member 236 is arranged in contact with the laser light source 211. As one example, in the light irradiation system 1 shown in FIG. 6, the heat-dissipating member 236 is arranged over a wide area of the surface of the laser light source 211 opposite to the side from which laser light is emitted. For the heat-dissipating member 236, a member having high thermal conductivity (for example, at least one of a heat pipe, carbon nanotubes, ceramics (AIN, SiC, etc.), a metal plate (platinum, titanium, copper, etc.), and a torque wire) is employed. As a result, the heat emitted from the laser light source 211 is conducted to the heat-dissipating member 236 and is then dissipated to the surroundings. As described above, when a metal plate is used, the metal plate may be an alloy, or the surface of the metal plate may be plated.

Furthermore, the heat-dissipating member 236 shown in FIG. 6 is exposed to the outside from a side surface of the device body 210 of the light irradiation device 2. Therefore, the heat-dissipating member 236 is exposed to the cooling liquid supplied into the lumen 311 of the catheter 3 (the space between the outer peripheral surface of the light irradiation device 2 and the inner peripheral surface of the lumen 311 of the catheter 3). Accordingly, the heat emitted from the laser light source 211 can be readily and appropriately released to the outside through the heat-dissipating member 236. It is more desirable that the heat-dissipating member 236 be in contact with the laser light source 211. However, even if the heat-dissipating member 236 is not in contact with the laser light source 211, the effect of suppressing a temperature rise of the laser light source 211 can be obtained as long as the heat-dissipating member 236 is close to the laser light source 211.

In the light irradiation system 1 according to the third modification shown in FIG. 7, unlike the other embodiments, the discharge valve 322 at the discharge port 341 of the catheter 3 is not used. However, in the light irradiation system 1 shown in FIG. 7, a discharge valve 238 is provided at a discharge port 221 formed at a distal end portion of the light irradiation device 2. The discharge valve 238 allows discharge of liquid from the inside to the outside of the cooling liquid passage 213 through the discharge port 341, while preventing inflow of liquid from the outside into the cooling liquid passage 213. As a result, the discharge valve 238 appropriately suppresses blood or the like outside the cooling liquid passage 213 from flowing into the inside through the discharge port 221.

It is also possible to employ only a part of the configurations exemplified in the above embodiments and modifications in a light irradiation system, a light irradiation device, or a catheter. For example, only one of the cooling liquid passage 213 and the Peltier element 230 may be employed in the light irradiation device. It is also possible to combine a plurality of configurations shown in different embodiments. As described above, it is also possible to use only the light irradiation device 2 alone without using the catheter 3.

The technology according to the first aspect of the present disclosure can also be expressed as follows.
(1) A medical light irradiation device having an elongated shape, comprising:
   a laser light source disposed at a distal end portion of an elongated device body and configured to emit laser light; and
   a cooling liquid passage extending to the laser light source in the device body and supplying a cooling liquid for cooling the laser light source to the laser light source.
(2) The light irradiation device according to (1), wherein
   the device body is formed in an elongated tubular shape to define the cooling liquid passage in the lumen of the device body, and
   when a cross-sectional area of the cooling liquid passage taken along a direction perpendicular to an extending direction of the device body is defined as a passage area, the passage area of the cooling liquid passage at a portion located on a proximal side of the laser light source is larger than the passage area of the cooling liquid passage at a portion where the laser light source is disposed.
(3) The light irradiation device according to (1) or (2), further comprising
   a power supply line extending from a proximal end side to a distal end side of the device body and supplying power to the laser light source by being connected to the laser light source, wherein
   the power supply line is exposed in the cooling liquid passage.
(4) The light irradiation device according to (3), wherein
   a cross-sectional area of a connecting portion where the power supply line is connected to the laser light source is larger than a cross-sectional area of a portion of the power supply line located on a proximal side of the connecting portion.
(5) The light irradiation device according to (3) or (4), wherein
   at least a part of a surface of the power supply line is covered with an insulating material, and
   a thickness of the insulating material covering the power supply line is smaller than a thickness of the power supply line without being covered with the insulating material.
(6) The light irradiation device according to any one of (1) to (5), further comprising:
   a supply detection unit configured to detect whether the cooling liquid is supplied into the cooling liquid passage; and
   a supply notification unit configured to provide notification of a detection result by the supply detection unit.
(7) The light irradiation device according to any one of (1) to (6), further comprising:
   a temperature detection unit configured to detect a temperature of the laser light source; and
   a temperature notification unit configured to provide notification of a detection result by the temperature detection unit.
(8) The light irradiation device according to any one of (1) to (7), further comprising
   a passage valve disposed in the cooling liquid passage on a proximal side of the laser light source to prevent reverse flow of the cooling liquid toward the proximal side.
(9) The light irradiation device according to any one of (1) to (8), further comprising a Peltier element disposed at a distal end portion of the device body, wherein the Peltier element is in contact with or in proximity to the laser light source and has a cooling surface facing the laser light source.
(10) The light irradiation device according to (9), wherein
   in the Peltier element, at least one of a heating surface located opposite to the cooling surface and an indirect heat-dissipating member having a thermal conductivity equal to or higher than that of the heating surface and being in contact with the heating surface is externally exposed to a liquid.
(11) A medical light irradiation system comprising:
   a catheter formed in an elongated tubular shape; and
   an elongated light irradiation device inserted into a lumen of the catheter, wherein
   the light irradiation device includes:
      a laser light source disposed at a distal end portion of an elongated device body and emitting laser light;
      a cooling liquid passage extending to the laser light source in the device body and supplying a cooling liquid for cooling the laser light source to the laser light source, and
      a light-transmitting portion that transmits, to the outside of the catheter, laser light emitted by the laser light source in the light irradiation device is formed in at least a part of the distal end portion of the catheter.
(12) The light irradiation system according to (11), wherein
   a discharge port for discharging, to the outside of the catheter, the cooling liquid discharged from the cooling liquid passage of the light irradiation device is formed in at least a part of the distal end portion of the catheter.
(13) The light irradiation system according to (12), wherein
   a discharge valve configured to allow discharge of liquid to the outside of the catheter through the discharge port while preventing inflow of liquid into the catheter from the outside is disposed at the discharge port of the catheter.

The technology according to the second aspect of the present disclosure can also be expressed as follows.
(1) A light irradiation device for medical use having an elongated shape, comprising:
   a laser light source disposed at a distal end portion of an elongated device body and emitting laser light; and
   a Peltier element disposed at the distal end portion of the device body, wherein the Peltier element is in contact with or in proximity to the laser light source and has a cooling surface facing the laser light source.
(2) The light irradiation device according to (1), wherein
   in the Peltier element, at least one of a heating surface located on a side opposite to the cooling surface and an indirect heat-dissipating member having thermal conductivity equal to or greater than that of the heating surface and being in contact with the heating surface is externally exposed to a liquid.
(3) The light irradiation device according to (1) or (2), further comprising
   a cooling liquid passage extending to the laser light source in the device body and supplying a cooling liquid for cooling at least one of the laser light source and the Peltier element to the laser light source.
(4) The light irradiation device according to (3), wherein
   the device body is formed in an elongated tubular shape to define the cooling liquid passage in a lumen of the device body, and
   when a cross-sectional area of the cooling liquid passage taken along a direction perpendicular to a longitudinal direction of the device body is defined as a passage area, the passage area of the cooling liquid passage at a portion of the device body located on a proximal side of the laser light source is larger than the passage area of the cooling liquid passage at a portion of the device body where the laser light source is disposed.
(5) The light irradiation device according to (3) or (4), further comprising
   a power supply line extending from a proximal end side to a distal end side of the device body and connected to the laser light source to supply electric power to the laser light source, wherein
   the power supply line is exposed in the cooling liquid passage.
(6) The light irradiation device according to (5), wherein
   a cross-sectional area of a connecting portion of the power supply line where the power supply line is connected to the laser light source is larger than a cross-sectional area of a portion of the power supply line located on a proximal side of the connecting portion.
(7) The light irradiation device according to (5) or (6), wherein
   at least a portion of a surface of the power supply line is covered with an insulating material, and
   a thickness of the insulating material covering the power supply line is smaller than a thickness of the power supply line without being covered with the insulating material.
(8) The light irradiation device according to any one of (3) to (7), further comprising:
   a supply detection unit configured to detect whether the cooling liquid is being supplied into the cooling liquid passage; and
   a supply notification unit configured to notify a detection result by the supply detection unit.
(9) The light irradiation device according to any one of (1) to (8), further comprising:
   a temperature detection unit configured to detect a temperature of the laser light source; and
   a temperature notification unit configured to notify a detection result by the temperature detection unit.
(10) The light irradiation device according to any one of (3) to (9), further comprising a passage valve disposed in the cooling liquid passage on a proximal side of the laser light source and configured to prevent reverse flow of the cooling liquid toward the proximal side of the cooling liquid passage.
(11) A light irradiation system for medical use, comprising:
   a catheter formed in an elongated tubular shape; and
   an elongated light irradiation device inserted into a lumen of the catheter, wherein the light irradiation device includes:
      a laser light source disposed at a distal end portion of an elongated device body and configured to emit laser light; and
      a Peltier element disposed at the distal end portion of the device body,
      the Peltier element is in contact with or in proximity to the laser light source and has a cooling surface facing the laser light source, and
      a light-transmitting portion that transmits, to an outside of the catheter, laser light emitted by the laser light source in the light irradiation device is formed in at least a part of a distal end portion of the catheter.
(12) The light irradiation system according to (11), wherein
   at least one of a heating surface opposite to the cooling surface of the Peltier element and an indirect heat-dissipating member having a thermal conductivity equal to or greater than that of the heating surface and being in contact with the heating surface is externally exposed to liquid .
(13) The light irradiation system according to (11) or (12), wherein
   the cooling liquid flows into the lumen of the catheter that is inserted into the light irradiation device.

## Claims

1. A light irradiation device for medical use having an elongated shape, comprising:
a laser light source disposed at a distal end portion of an elongated device body and configured to emit laser light; and
a cooling liquid passage extending to the laser light source in the device body and supplying a cooling liquid for cooling the laser light source to the laser light source.

2. The light irradiation device according to claim 1, wherein
the device body is formed in an elongated tubular shape defining the cooling liquid passage in a lumen of the device body, and
when a cross-sectional area of the cooling liquid passage taken along a direction perpendicular to a longitudinal direction of the device body is defined as a passage area, the passage area of the cooling liquid passage at a portion of the device body located on a proximal side of the laser light source is larger than the passage area of the cooling liquid passage at a portion of the device body where the laser light source is disposed.

3. The light irradiation device according to claim 1, further comprising
a power supply line extending from a proximal end side to a distal end side of the device body and connected to the laser light source to supply power to the laser light source, wherein
the power supply line is exposed in the cooling liquid passage.

4. The light irradiation device according to claim 3, wherein
a cross-sectional area of a connecting portion of the power supply line where the power supply line is connected to the laser light source is larger than a cross-sectional area of a portion of the power supply line located on a proximal side of the connecting portion.

5. The light irradiation device according to claim 3, wherein
at least a portion of a surface of the power supply line is covered with an insulating material, and
a thickness of the insulating material covering the power supply line is smaller than a thickness of the power supply line without being covered with the insulating material.

6. The light irradiation device according to claim 1, further comprising:
a supply detection unit configured to detect whether cooling liquid is being supplied into the cooling liquid passage; and
a supply notification unit configured to notify a detection result by the supply detection unit.

7. The light irradiation device according to claim 1, further comprising:
a temperature detection unit configured to detect a temperature of the laser light source; and
a temperature notification unit configured to notify a detection result by the temperature detection unit.

8. The light irradiation device according to claim 1, further comprising
a passage valve disposed in the cooling liquid passage on a proximal side of the laser light source and configured to prevent reverse flow of the cooling liquid toward the proximal side of the cooling liquid passage.

9. The light irradiation device according to claim 1, further comprising
a Peltier element disposed at the distal end portion of the device body, wherein
the Peltier element is in contact with or in proximity to the laser light source and has a cooling surface facing the laser light source.

10. The light irradiation device according to claim 9, wherein
at least one of a heating surface opposite to the cooling surface of the Peltier element and an indirect heat-dissipating member having a thermal conductivity equal to or greater than that of the heating surface and being in contact with the heating surface is externally exposed to liquid .

11. A light irradiation device for medical use having an elongated shape, comprising:
a laser light source disposed at a distal end portion of an elongated device body and configured to emit laser light; and
a Peltier element disposed at the distal end portion of the device body, wherein
the Peltier element is in contact with or in proximity to the laser light source and has a cooling surface facing the laser light source.

12. A light irradiation system for medical use, comprising:
a catheter formed in an elongated tubular shape; and
an elongated light irradiation device inserted into a lumen of the catheter, wherein
the light irradiation device includes:
a laser light source disposed at a distal end portion of an elongated device body and configured to emit laser light; and
a cooling liquid passage extending to the laser light source in the device body and supplying a cooling liquid for cooling the laser light source to the laser light source, and
a light-transmitting portion configured to transmit, to an outside of the catheter, laser light emitted by the laser light source in the light irradiation device is formed in at least a part of a distal end portion of the catheter.

13. The light irradiation system according to claim 12, wherein
a discharge port for discharging, to an outside of the catheter, the cooling liquid discharged from the cooling liquid passage of the light irradiation device is formed in at least a part of the distal end portion of the catheter.

14. The light irradiation system according to claim 13, wherein
a discharge valve configured to allow discharge of liquid to the outside of the catheter through the discharge port while preventing inflow of liquid into the catheter from the outside is disposed at the discharge port of the catheter.

15. A light irradiation system for medical use, comprising:
a catheter formed in an elongated tubular shape; and
an elongated light irradiation device inserted into a lumen of the catheter, wherein
the light irradiation device includes:
a laser light source disposed at a distal end portion of an elongated device body and configured to emit laser light; and
a Peltier element disposed at the distal end portion of the device body,
the Peltier element is in contact with or in proximity to the laser light source and has a cooling surface facing the laser light source, and
a light-transmitting portion transmitting, to an outside of the catheter, the laser light emitted by the laser light source in the light irradiation device is formed in at least a part of a distal end portion of the catheter.
